# EUROPEAN PATENT SPECIFICATION

(11) **EP 2 118 058 B1**
(45) Date of publication and mention of the grant of the patent: **08.06.2011**
(21) Application number: 08706720.3
(22) Date of filing: 11.02.2008
(51) Int. Cl.: C07D 209/12, C07C 217/80

(54) **METHOD FOR THE PREPARATION OF 5-BENZYLOXY-2-(4-BENZYLOXPHENYL)-3-METHYL-1H-INDOLE**
VERFAHREN ZUR HERSTELLUNG VON 5-BENZYLOXY-2-(4-BENZYLOXYPHENYL)-3-METHYL-1H-INDOL
PROCÉDÉ D'ÉLABORATION DE 5-BENZYLOXY-2-(4-BENZYLOXPHÉNYL)-3-MÉTHYL-1H-INDOLE

(30) Priority: 12.02.2007 CZ 20070110
(43) Date of publication of application: 18.11.2009
(73) Proprietor: Zentiva, k.s., Dolni Mecholupy 102 37 Praha 10 (CZ)
(72) Inventor: JIRMAN, Josef, 100 00 Praha 10 (CZ); RICHTER, Jindrich, 530 12 Pardubice (CZ)
(74) Representative: Jirotkova, Ivana
(86) International application number: PCT/CZ2008/000016
(87) International publication number: WO 2008/098527

(56) References cited:
- EP-A- 0 802 183
- WO-A-99/19293

## Description

### Technical Field

The invention deals with a new method of preparation of 5-benzyloxy-2-(4-benzyloxyphenyl)-3-methyl-1*H*-indole of formula 1 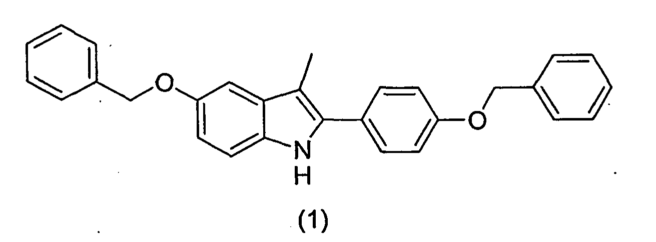 which is used for the production of 2-(4-hydroxyphenyl)-1-[4-(2-azepan-1-yl-ethoxy)benzyl]-3-methyl-1*H*-indol-5-ol (bazedoxifen) of formula 2.

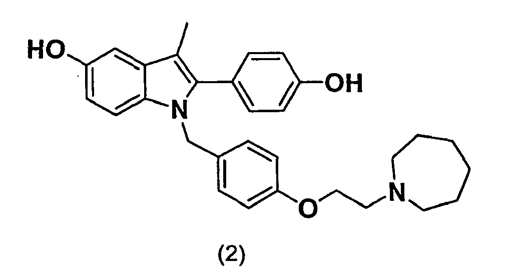

Bazedoxifen is an agonist of oestrogen; it is used within hormone substitution therapy for prevention of bone tissue loss, replacement of oestrogen and prevention of heart and vein diseases in post-menopausal women.

### Background Art

In literature two analogous methods for the preparation of bazedoxifen have been found - see Scheme 1. The methods differ in the protective group (PG - methyl, benzyl), in the way of preparation and connection of the chain to the nitrogen of the indole heterocycle and the way of removal of the protective group.

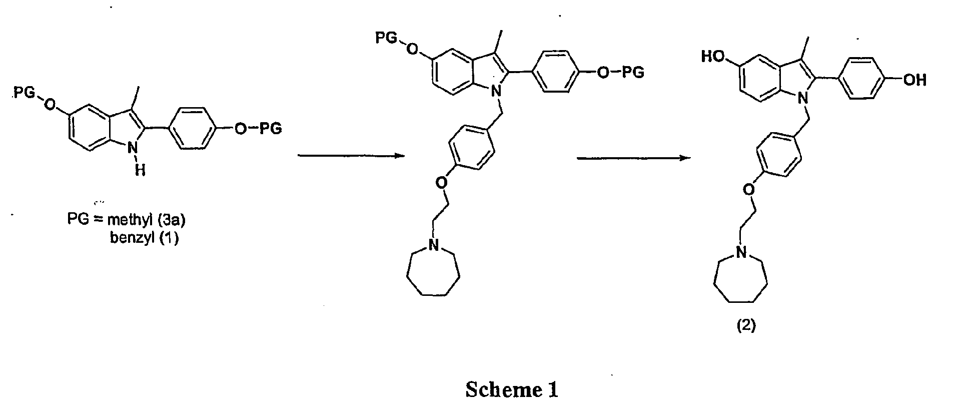

5-Methoxy-2-(4-methoxyphenyl)-3-methyl-1*H*-indo of formula 3a is prepared by the Bischler method (J.Med.Chem. 1984, 27, 1439-1447; WO 9603375) from 2-bromo-4'-methoxypropiophenone of formula 5 and *p*-anisidine of formula 6 in o-xylene in the presence of N,N-dimethylaniline. 2-Bromo-4'-methoxypropiophenone of formula 5 is obtained by bromination of 4-methoxypropiophenone of formula 4 with bromine in acetic acid (WO 9603375), see Scheme 2.

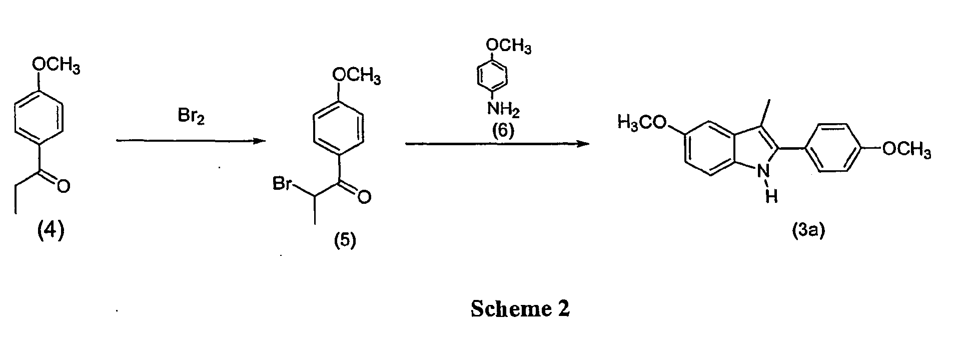

The total yield of the two-stage synthesis is 22 %.

5-Benzyloxy-2-(4-benzyloxyphenyl)-3-methyl-1*H*-indole of formula 1 is also prepared by the Bischler method from 2-bromo-4'-benzyloxypropiophenone of formula 8 and 4-benzyloxyaniline hydrochloride of formula 9 in N,N-dimethylformamide (EP 0802183). 2-Bromo-4'-benzyloxypropiophenone of formula 8 is obtained by bromination of 4-benzyloxypropiophenone of formula 7 with bromine in acetic acid. The total yield of the two-stage synthesis (see Scheme 3) is 47%.

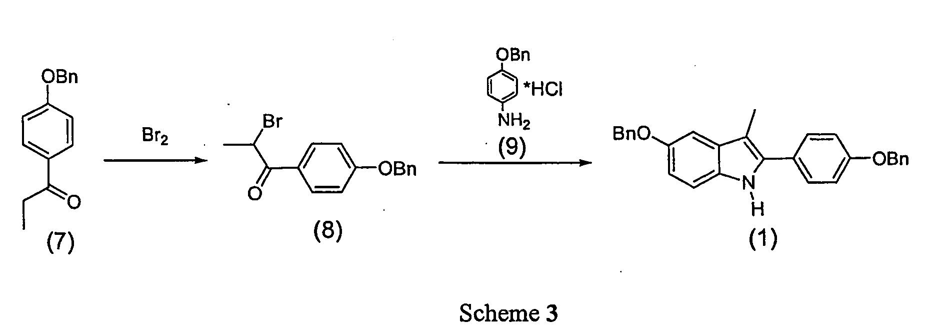

Patent application no. WO 9919293 mentions carrying the second stage out in toluene with N,N-diisopropylethylamine under reflux; however, without any further specification in the examples or reference to literature.

Verification syntheses have shown that with the above mentioned preparation methods the compound of formula 1 cannot be obtained with a sufficiently high yield and, especially, in sufficient purity.

In reproducing the process in accordance with patent no. EP 802183 (Scheme 4) it has been found out that during this synthesis high quantities of undesired substances are generated.

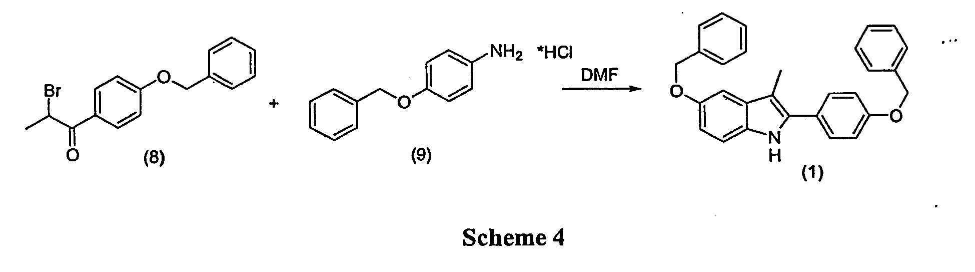

Isolation of the compound of formula 1 prepared this way is considerably complicated and its purification significantly reduces the yield of the synthesis.

In carrying the reaction out in accordance with the patent application no. WO 9919293, which describes preparation of the compound of formula 1 in the environment of toluene and N,N-diisopropylethylamine under simultaneous removal of water by azeotropic distillation, it has been found out that the reaction time takes several tens of hour. During this time already a substantial amount of secondary substances are generated that reduce the yield and quality of the product.

For this reason there was an effort to find a more efficient way of synthesis, the result of which is the new method for the preparation of 5-benzyloxy-2-(4-benzyloxyphenyl)-3-methyl-1*H-*indole of formula 1, which consists the object of the present invention.

### Disclosure of Invention

The invention deals with a new method for the preparation of 5-benzyloxy-2-(4-benzyloxyphenyl)-3-methyl-1*H*-indole of formula 1, which is based on isolation of the intermediate *N*-(4-benzyloxyphenyl)-α-amino-4-benzyloxypropiophenone of formula 10. This intermediate product is preferably obtained by reaction of 4-benzyloxyaniline or its salt with a substance of formula 11 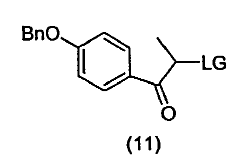 wherein LG is a leaving group, e.g. Cl, Br, I, alkylsufonyl or arylsulfonyl.

The preparation process comprises
a) Reaction of 2-bromo-4'-benzyloxypropiophenone of formula 8 with *p*-benzyloxyaniline hydrochloride of formula 9, the reaction being carried out in the environment of an organic solvent from the group of C₁ to C₄ alcohols, toluene, acetone, methyltetrahydrofuran and in the presence of an inorganic or organic base from the group including sodium carbonate, potassium carbonate, DIPEA and triethylamine; 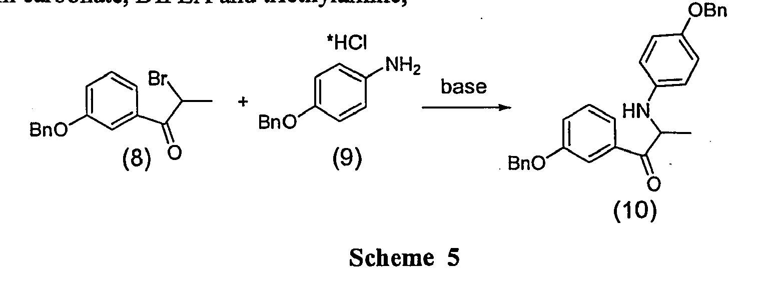
b) Isolation of *N*-(4-benzyloxyphenyl)-α-amino-4-benzyloxypropiophenone of formula 10 in the solid state;
c) Cyclization of *N*-(4-benzyloxyphenyl)-α-amino-4-benzyloxypropiophenone of formula 10 by reaction with *p*-benzyloxyaniline hydrochloride of formula 9 in the environment of a suitable organic solvent from the group of C₁ to C₄ alcohols, toluene, acetone, methyltetrahydrofuran to give 5-benzyloxy-2-(4-benzyloxyphenyl)-3-methyl-1*H*-indole of formula 1 in the solid state.

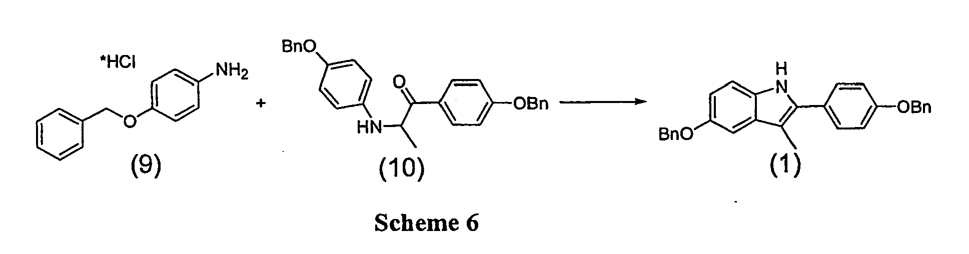

It has been found out that it is advantageous to carry out synthesis of 5-benzyloxy-2-(4-benzyloxyphenyl)-3-methyl-1H-indole of formula 1 in 2 stages with isolation of the intermediate of formula 10 in accordance with Scheme 7.

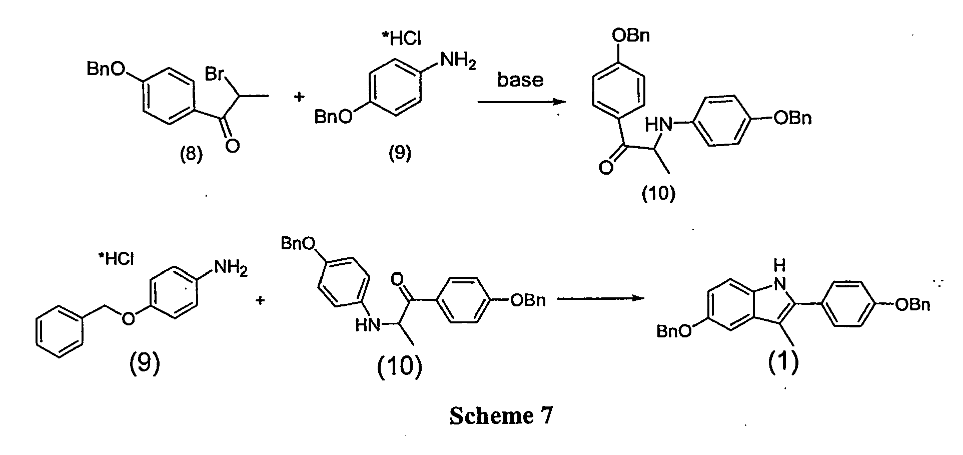

In the first stage the starting compounds 8 and 9 are reacted in the environment of an organic solvent from the group of C₁ to C₄ alcohols, toluene, ketones, methyltetrahydrofuran, preferably ethanol, and an inorganic or organic base from the group including sodium carbonate, triethylamine and DIPEA, preferably triethylamine, at the reflux temperature for 4-6 hours. In this manner a suspension of the intermediate 10 and possibly inorganic salts (corresponding to the base used) is formed after several hours. The intermediate is isolated by filtration. The product yield is 81 to 100 %.

The product can be re-crystallized by dissolution in a mixture of a polar and non-polar solvent (ethyl acetate-ethanol, toluene-methanol, THF-methanol).

In the second stage *N*-(4-benzyloxyphenyl)-α-amino-4-benzyloxypropiophenone of formula 10 is suspended, together with *p*-benzyloxyaniline hydrochloride of formula 9 (molar ratio 1:20 to 1:1, preferably 1:5 with respect to the compound of formula 10), in an organic solvent from the group of C₁-C₄ alcohols, toluene, and methyltetrahydrofuran, preferably ethanol, and the mixture is heated up in a pressure vessel under inert atmosphere to 100 to 120°C. After several hours (4 to 5) the reaction mixture is cooled to the laboratory temperature. The crystallized product 5-benzyloxy-2-(4-benzyloxyphenyl)-3-methyl-1*H*-indole of formula 1 is filtered, washed with ethanol and optionally re-crystallized from a mixture of a polar and non-polar solvent (ethyl acetate-ethanol, toluene-methanol, THF-methanol). The yield of the reaction is 75 to 80%.

It is advantageous that the starting aniline of formula 9 can be obtained from the mother liquor, after concentrating and stirring the concentrated matter up in ethyl acetate, back with nearly 100% yield.

This original method is based on the preparation and isolation of a new substance, the intermediate *N*-(4-benzyloxyphenyl)-α-amino-4-benzyloxypropiophenone of formula 10. The main advantages of the method include a higher yield (60 to 75%) as compared to the published methods (35 to 53 %), easy isolation of the intermediate *N*-(4-benzyloxyphenyl)-α-amino-4-benzyloxypropiophenone of formula 10 as well as the final product 5-benzyloxy-2-(4-benzyloxyphenyl)-3-methyl-1*H*-indole of formula 1 by filtration directly from the reaction mixture without using any additional chemicals and, above all, the achievement of a high quality of the crude product already (HPLC 98 to 100 %). Another advantage is that the starting benzyloxyaniline hydrochloride of formula 9, used for the cyclization reaction, is not consumed and can be obtained from the mother liquors after isolation of 5-benzyloxy-2-(4-benzyloxyphenyl)-3-methyl-1*H*-indole of formula 1 in a quantitative yield.

### Brief Description of Drawings

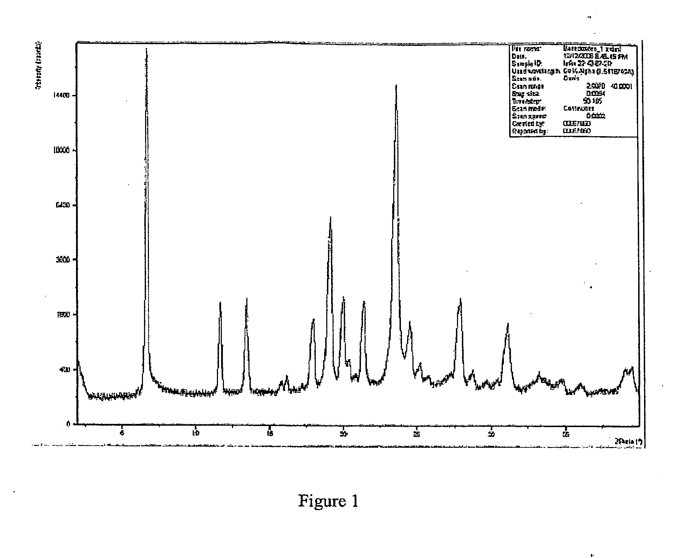

Figure 1 represents an X-ray diffraction pattern of *N*-(4-benzyloxyphenyl)-α-amino-4-benzyloxypropiophenone of formula 10 (conditions of the X-ray analysis: X'Pert PRO PANalytical diffractometer, CuKa radiation (1= 1.542 Å) in the range of 4 - 40° 2θ with the increment of 0.008).

### Examples

The invention is described in more detail in the following examples.

### Preparation of N-(4-benzyloxyphenyl)-α-amino-4-benzyloxypropiophenone (10)

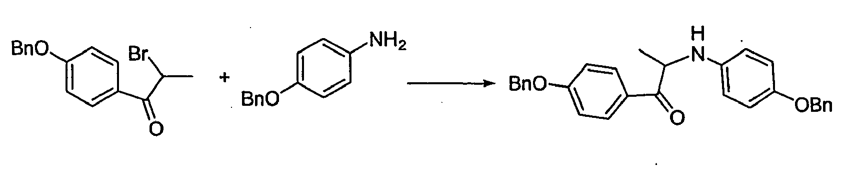

1) α-Bromo-4-benzyloxypropiophenone (20 g; 62.7 mmol), 4-benzyloxyaniline (16 g; 67.9 mmol) and triethylamine (19 ml; 136.4 mmol) were suspended in 250 ml of toluene and the mixture was refluxed for 5 hours. Then, the reaction mixture was filtered and concentrated to ca. 1/3 of the volume. Ethanol (80 ml) was added to the concentrated matter and the mixture was cooled to 5°C. With filtration 19.2 g (71 %) of a grey product, *N*-(4-benzyloxyphenyl)-α-amino-4-benzyloxypropiophenone (10), were obtained with the melting temperature of 124.5-126°C.
2) α-Bromo-4-benzyloxypropiophenone (26 g; 81.5 mmol), 4-benzyloxyaniline (23 g; 97.8 mmol) and sodium carbonate (20.7 g; 196 mmol) were suspended in 300 ml of ethanol and the mixture was refluxed for 5 hours. During said period the product precipitates. After cooling of the reaction mixture a mixture of the salts (NaBr, NaCl, sodium carbonate) and the crystallized product was isolated by filtration. The isolated mixture of substances was dissolved in a toluene-water mixture. After separation of the aqueous phase the organic phase was concentrated. Ethanol was added to the concentrated residue. The precipitated slightly yellowish crystalline product was isolated by filtration and washed with ethanol. 30.2 g (85%) of *N*-(4-benzyloxyphenyl)-α-amino-4-benzyloxypropiophenone (10) were obtained. Melting temperature 126.0-127.1°C.
3) α-Bromo-4-benzyloxypropiophenone (16.1 g; 50.5 mmol), 4-benzyloxyaniline (14.2 g; 60.1 mmol) and triethylamine (16.1 ml; 115.6 mmol) were suspended in 130 ml of ethanol and the mixture was refluxed for 5 hours. Then, being stirred the mixture was cooled to the laboratory temperature within 1 hour. The filtered product was washed with ethanol and dried. 21.9 g (99.5 %) of *N*-(4-benzyloxyphenyl)-α-amino-4-benzyloxypropiophenone (10) were obtained. Melting temperature 122.6-125.4°C.

The crude product, *N*-(4-benzyloxyphenyl)-α-amino-4-benzyloxypropiophenone (10) (15.4 g) was dissolved in toluene (40 ml) by heating up to 60°C. The solution was filtered and ethanol (40 ml) was added to the filtrate. After cooling to 10 to 15°C 13.8 g (89.6%) of a white product with the melting temperature of 126.1-127.1°C were obtained. ¹H-NMR (DMSO) δ 8.10 (d, 2H); 7.3-7.5 (m, 12H); 7.19 (d, 2H); 7.06 (d, 2H); 5.5 (q, 1H); 5.24 (s, 2H); 5.08 (s, 2H); 1.48 (d, 3H).

The X-ray difractogram of the obtained product is shown in figure 1; values of the characteristic diffraction angles 2θ: 6.71; 19.00, 19.13; 23.49; 23.63.

### Preparation of 5-benzyloxy-2-(4-benzyloxyphenyl)-3-methyl-1H-indole (1)

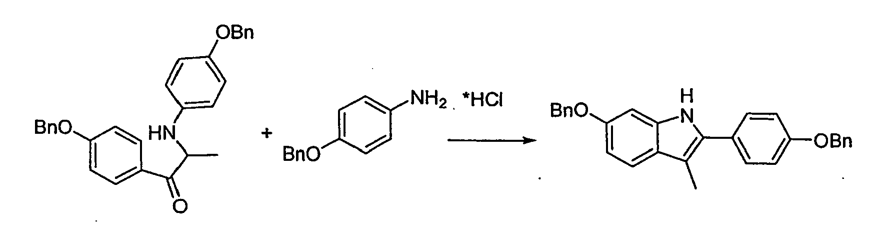

1) The starting *N*-(4-benzyloxyphenyl)-α-amino-4-benzyloxypropiophenone (22) (30.4 g; 69.7 mmol) and 4-benzyloxyaniline hydrochloride (3.3 g; 13.9 mmol) were suspended in ethanol (380 ml) and the mixture was heated to 110-115°C under an inert atmosphere in a pressure vessel. After 5 hours the heating was disconnected and the reaction mixture was stirred overnight. The crystallized white product was filtered and washed with ethanol. 23.3 g (79%) of 5-benzyloxy-2-(4-benzyloxyphenyl)-3-methyl-1*H*-indole (3b) with the melting temperature of 152.4-153.4°C were obtained. ¹H-NMR (DMSO) δ 10.65 (s, 1H); 7.55 (d, 2H); 7.50 (d, 4H); 7.30-7.45 (m, 6H); 7.21(d, 1H); 7.10 (d, 2H); 7.10 (d, 1H); 6.91 (dd, 1H); 5.16 (s, 2H); 5.11 (s, 2H); 2.33 (s, 3H). MS eI m/z 419.
2) The starting *N*-(4-benzyloxyphenyl)-α-amino-4-benzyloxypropiophenone (22) (30.4 g; 69.7 mmol) and 4-benzyloxyaniline hydrochloride (3.3 g; 13.9 mmol) were suspended in propan-2-ol (380 ml) and the mixture was heated up to 110-115°C under an inert atmosphere in a pressure vessel. After 5 hours the heating was disconnected and the reaction mixture was stirred overnight. The crystallized beige product was filtered and washed with a small quantity of propan-2-ol. 24.2 g (82 %) of 5-benzyloxy-2-(4-benzyloxyphenyl)-3-methyl-1*H*-indole (3b) with the melting temperature of 152.0-153.2°C were obtained.

The mother liquors after the filtration were concentrated and the concentrated matter was stirred up in ethyl acetate (30 ml). The precipitated crystalline substance was filtered. 3.3 g of beige 4-benzyloxyaniline hydrochloride were obtained.

## Claims

1. A method for the preparation of 5-benzyloxy-2-(4-benzyloxyphenyl)-3-methyl-1*H-*indole of formula 1 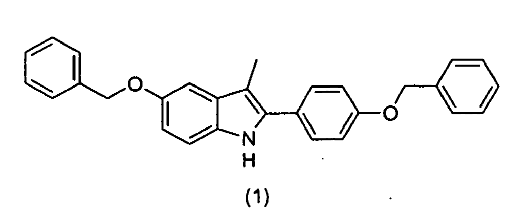 **characterized in that** it comprises isolation of the intermediate *N*-(4-benzyloxyphenyl)-α-amino-4-benzyloxypropiophenone of formula 10 in the solid state.

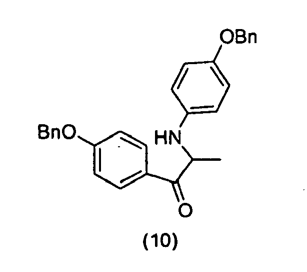

2. The method according to claim 1, **characterized in that** the intermediate of formula 10 is obtained by a reaction of 4-benzyloxyaniline or its salt and the substance of formula 11 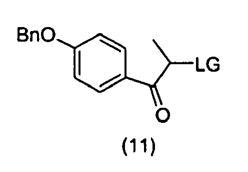 wherein LG is a leaving group, e.g. Cl, Br, I, alkylsulfonyl or arylsulfonyl.

3. The method according to claim 1 and 2, **characterized in that** the reaction of 2-bromo-4'-benzyloxypropiophenone of formula 8 with *p*-benzyloxyaniline hydrochloride of formula 9 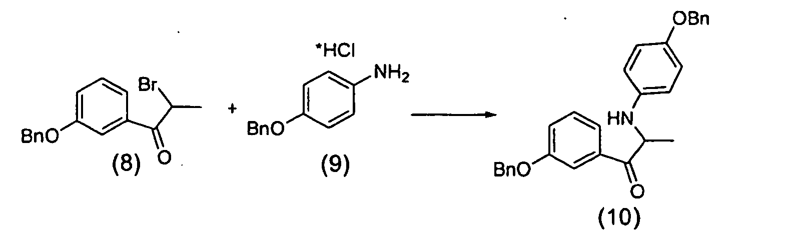 is carried out in the environment of an organic solvent and in the presence of an inorganic or organic base.

4. The method according to claims 1 to 3, **characterized in that** the reaction is carried out in the environment of an organic solvent from the group of C₁ to C₄ alcohols, toluene, acetone, methyltetrahydrofuran and in the presence of an inorganic or organic base from the group including sodium carbonate, potassium carbonate, triethylamine, diisopropylethylamine.

5. The method according to claim 4, **characterized in that** ethanol is used as the solvent and triethylamine as the base.

6. The method according to claims 3 to 4, **characterized in that** the reaction is carried out under reflux.

7. The method according to claims 1 to 6, **characterized in that** the intermediate *N*-(4-benzyloxyphenyl)-α-amino-4-benzyloxypropiophenone is further purified by crystallization from an organic solvent selected from the group of liquid C₁ to C₁₅ aliphatic, alicyclic or aromatic hydrocarbons, or their oxidation or nitrogen-containing derivatives, or their mixtures.

8. The method according to claim 7, **characterized in that** for the crystallization of the intermediate a mixture of a polar and non-polar solvent is used, such as mixtures ethyl acetate-ethanol, toluene-methanol, THF-methanol.

9. The method according to claims 1 and 2, **characterized in that** 5-benzyloxy-2-(4-benzyloxyphenyl)-3-methyl-1*H*-indole of formula 1 is prepared by cyclization of *N-*(4-benzyloxyphenyl)-α-amino-4-benzyloxypropiophenone of formula 10, prepared by the method according to claims 3 to 8, by action of *p*-benzyloxyaniline hydrochloride of formula 9 in the environment of an organic solvent from the group of C₁ to C₄ alcohols, toluene, acetone and methyltetrahydrofuran.

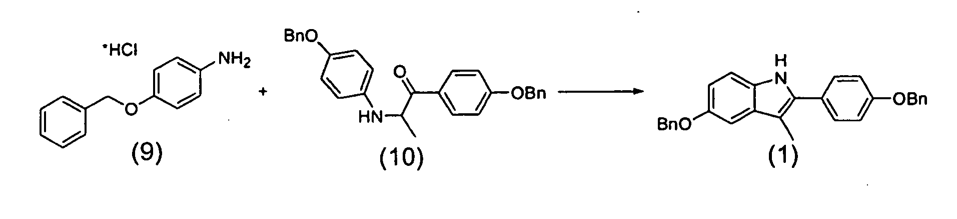

10. The method according to claim 9, **characterized in that** p-benzyloxyaniline hydrochloride of formula 9 is used in a molar ratio of 1:20 to 1:1, preferably 1:5, with respect to the compound of formula 10.

11. The method according to claims 9 to 10, **characterized in that** the reaction is carried out in a pressure vessel under inert atmosphere at an increased temperature of 100 to 120°C.

12. The method according to claims 9 to 11, **characterized in that** an organic solvent from the group of C₁-C₄ alcohols, preferably ethanol, is used.

13. Crystalline N-(4-benzyloxyphenyl)-α-amino-4-benzyloxypropiophenone of formula 10.

14. The product according to claim 13, **characterized by** the following values of characteristic diffraction angles 2θ in the powder X-ray diffraction pattern (XRPD): 6.71; 19.00, 19.13; 23.49; 23.63.

15. Use of *N*-(4-benzyloxyphenyl)-α-amino-4-benzyloxypropiophenone of formula 10 for the preparation of 5-benzyloxy-2-(4-benzyloxyphenyl)-3-methyl-1*H*-indole of formula 1.

16. Use of *N*-(4-benzyloxyphenyl)-α-amino-4-benzyloxypropiophenone of formula 10 for the preparation of 2-(4-hydroxyphenyl)-1-[4-(2-azepan-1-yl-ethoxy)benzyl]-3-methyl-1H-indole-5-ol (bazedoxifen) of formula 2.

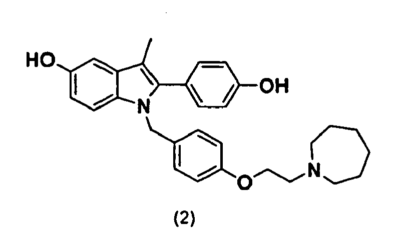

## Patentansprüche

1. Verfahren zur Herstellung von 5-Benzyloxy-2-(4-benzyloxyphenyl)-3-methyl-1H-indol der Formel (1) 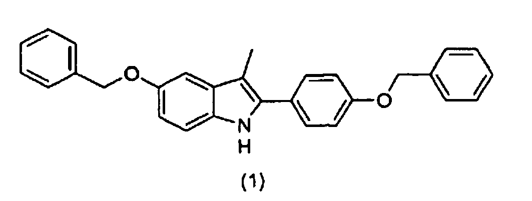 **dadurch gekennzeichnet, dass** es die Isolierung des Intermediats N-(4-Benzyloxyphenyl)-α-amino-4-benzyloxypropiophenon der Formel (10) in festem Zustand umfasst:

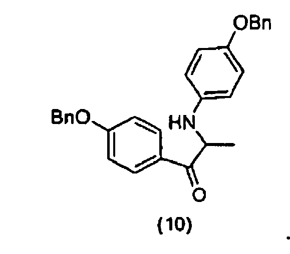

2. Verfahren gemäss Anspruch 1, **dadurch gekennzeichnet, dass** das Intermediat der Formel (10) durch Umsetzen von 4-Benzyloxyanilin oder seines Salzes und einer Substanz der Formel (11): 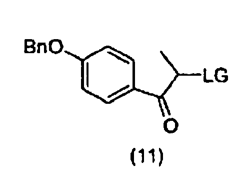 worin LG eine Abgangsgruppe, z.B. Cl, Br, I, Alkylsulfonyl oder Arylsulfonyl, ist, erhalten wird.

3. Verfahren gemäss Anspruch 1 oder 2, **dadurch gekennzeichnet, dass** die Umsetzung von 2-Brom-4'-benzyloxypropiophenon der Formel (8) mit p-Benzyloxyanilin-Hydrochlorid der Formel (9): 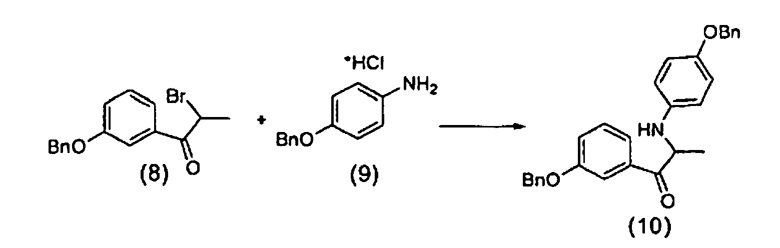 in der Umgebung eines organischen Lösungsmittels und in Gegenwart einer anorganischen oder organischen Base durchgeführt wird.

4. Verfahren gemäss Ansprüchen 1 bis 3, **dadurch gekennzeichnet, dass** die Umsetzung in der Umgebung eines organischen Lösungsmittels der Gruppe von C₁₋₄-Alkoholen, Toluol, Aceton und Methyltetrahydrofuran und in Gegenwart einer anorganischen oder organischen Base der Gruppe, umfassend Natriumcarbonat, Kaliumcarbonat, Triethylamin und Diisopropylethylamin, durchgeführt wird.

5. Verfahren gemäss Anspruch 4, **dadurch gekennzeichnet, dass** Ethanol als Lösungsmittel und Triethylamin als Base verwendet werden.

6. Verfahren gemäss Ansprüchen 3 bis 4, **dadurch gekennzeichnet, dass** die Umsetzung unter Rückfluss durchgeführt wird.

7. Verfahren gemäss Ansprüchen 1 bis 6, **dadurch gekennzeichnet, dass** das Intermediat N-(4-Benzyloxyphenyl)-α-amino-4-benzyloxypropiophenon ferner durch Kristallisation aus einem organischen Lösungsmittel, ausgewählt aus der Gruppe von flüssigen C₁₋₁₅ aliphatischen, alicyclischen oder aromatischen Kohlenwasserstoffen oder deren Oxidations- oder stickstoffhaltigen Derivaten oder deren Mischungen, gereinigt wird.

8. Verfahren gemäss Anspruch 7, **dadurch gekennzeichnet, dass** zur Kristallisation des Intermediats eine Mischung aus einem polaren und nicht-polaren Lösungsmittel, wie Ethylacetat-Alkohol-, Toluol-Methanol- und THF-Methanol-Mischungen, verwendet wird.

9. Verfahren gemäss Ansprüchen 1 und 2, **dadurch gekennzeichnet, dass** 5-Benzyloxy-2-(4-benzyloxyphenyl)-3-methyl-1H-indol der Formel (1) durch Cyclisieren von N-(4-Benzyloxyphenyl)-α-amino-4-benzyloxypropiophenon der Formel (10), hergestellt nach dem Verfahren gemäss Ansprüchen 3 bis 8, durch Einwirken von p-Benzyloxyanilin-Hydrochlorid der Formel (9) in einer Umgebung eines organischen Lösungsmittels aus der Gruppe von C₁₋₄-Alkoholen, Toluol, Aceton und Methyltetrahydrofuran hergestellt wird:

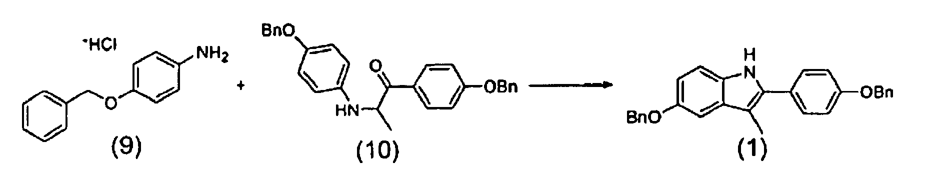

10. Verfahren gemäss Anspruch 9, **dadurch gekennzeichnet, dass** p-Benzyloxyanilin-Hydrochlorid der Formel (9) in einem molaren Verhältnis von 1:20 bis 1:1, vorzugsweise 1:5, in bezug auf die Verbindung der Formel (10), verwendet wird.

11. Verfahren gemäss Ansprüchen 9 bis 10, **dadurch gekennzeichnet, dass** die Umsetzung in einem Druckkessel unter inerter Atmosphäre bei einer erhöhten Temperatur von 100 bis 120°C durchgeführt wird.

12. Verfahren gemäss Ansprüchen 9 bis 11, **dadurch gekennzeichnet, dass** ein organisches Lösungsmittel aus der Gruppe von C₁₋₄-Alkoholen, vorzugsweise Ethanol, verwendet wird.

13. Kristallines N-(4-Benzyloxyphenyl)-α-amino-4-benzyloxypropiophenon der Formel (10).

14. Produkt gemäss Anspruch 13, **gekennzeichnet durch** die nachstehenden Werte der charakteristischen Beugungswinkel 2θ im Pulver-Röntgenbeugungsmuster (XRPD): 6,71, 19,00, 19,13, 23,49 und 23,63.

15. Verwendung von N-(4-Benzyloxyphenyl)-α-amino-4-benzyloxypropiophenon der Formel (10) zur Herstellung von 5-Benzyloxy-2-(4-benzyloxyphenyl)-3-methyl-1H-indol der Formel (1).

16. Verwendung von N-(4-Benzyloxyphenyl)-α-amino-4-benzyloxypropiophenon der Formel (10) zur Herstellung von 2-(4-Hydroxyphenyl)-1-[4-(2-azepam-1-yl-ethoxy)-benzyl]-3-methyl-1H-indol-5-ol (Bazedoxifen) der Formel (2):

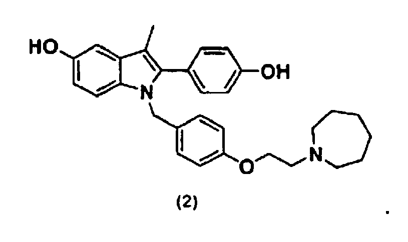

## Revendications

1. Un procédé pour la préparation de 5-benzyloxy-2-(4-benzyloxyphényl)-3-méthyl-1*H*-indole de formule: 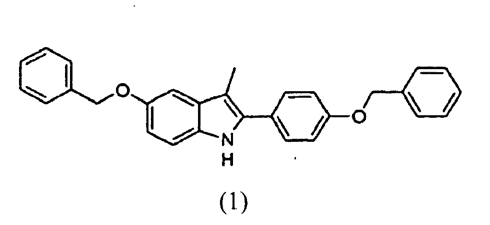 **caractérisé en ce qu'**il comprend l'isolation du *N*-(4-benzyloxyphényl)-α-amino-4-benzyloxy-propio-phénone intermédiaire de formule 10, à l'état solide.

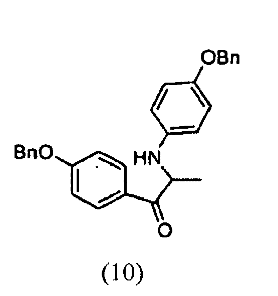

2. Le procédé selon la revendication 1, **caractérisé en ce que** l'intermédiaire de formule 10 est obtenu par une réaction entre la 4-benzyloxyaniline ou l'un de ses sels avec la substance de formule 11 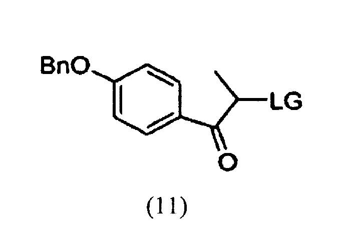 dans laquelle LG est un groupe partant, e.g. Cl, Br, I, alkylsulfonyl ou arylsulfonyl.

3. Le procédé selon les revendications 1 et 2, **caractérisé en ce que** la réaction de la 2-bromo-4'-benzyloxypropiophénone de fomule 8 avec le chlorhydrate de p-benzyloxyaniline de formule 9. 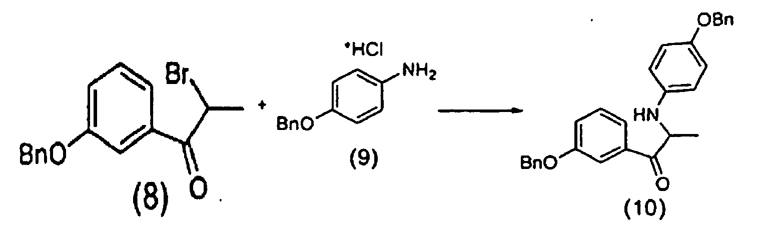 est effectuée au sein d'un solvant organique et en présence d'une base inorganique ou organique.

4. Le procédé selon les revendications 1 à 3, **caractérisé en ce que** la réaction est effectuée au sein d'un solvant organique sélectionné dans le groupe comprenant les alcools en C₁ à C₄, le toluène, l'acétone, le méthyltétrahydrofuranne en présence d'une base inorganique ou organique choisie dans le groupe comprenant le carbonate de sodium, le carbonate de potassium, la triéthylamine, la diisopropyléthylamine.

5. Le procédé selon la revendication 4, **caractérisé en ce que** l'éthanol est utilisé comme solvant et la triéthylamine comme base.

6. Le procédé selon les revendications 3 et 4, **caractérisé en ce que** la réaction est effectuée sous reflux.

7. Le procédé selon les revendications 1 à 6, **caractérisé en ce que** la N-(4-benzyloxyphényl)-α-amino-4-benzyloxypropiophénone intermédiaire, est en outre purifiée par cristallisation d'un solvant organique choisi dans le groupe formé par les hydrocarbures aliphatiques en C₁ à C₁₅ liquides, alicycliques ou aromatiques ou leurs dérivés obtenus par oxydation, leurs dérivés azotés ou leurs mélanges.

8. Le procédé selon la revendication 7, **caractérisé en ce que** pour la cristallisation de l'intermédiaire, est utilisé un mélange d'un solvant polaire et d'un solvant non polaire, tels des mélanges éthylacétate/éthanol, toluène/méthanol, THF/méthanol.

9. Le procédé selon les revendications 1 et 2, **caractérisé en ce que** le 5-benzyl-oxy-2(4-benzyloxyphényl)-3-méthyl-1*H*-indole de formule 1 est préparé par cyclisation de *N*-(4-benzyloxyphényl)-α-amino-4-benzyloxypropiophénone de formule 10, préparé par le procédé selon les revendications 3 à 8, par action de chlorhydrate de p-benzyloxyaniline de formule 9 au sein d'un solvant organique sélectionné dans le groupe comprenant les alcools en C₁ à C₄, le toluène, l'acétone, le méthyltétrahydrofuranne.

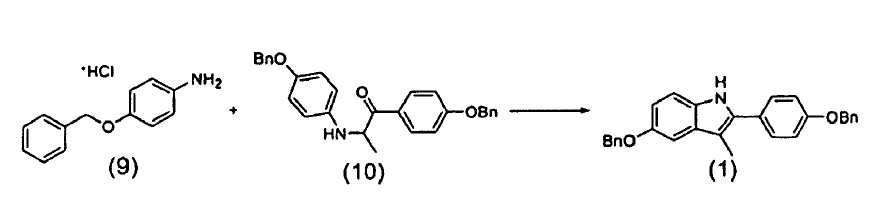

10. Le procédé selon la revendication 9, **caractérisé en ce que** chlorhydrate de p-benzyloxyaniline de formule 9 est utilisé en un rapport molaire compris entre 1/20 et 1/1, de préférence de 1:5, exprimé par rapport au composé de formule 10.

11. Le procédé selon les revendications 9 à 10, **caractérisé en ce que** la réaction est effectué dans u récipient sous pression sous atmosphère inerte à une température augmentée de 100 à 120°C.

12. Le procédé selon les revendications 9 à 11, **caractérisé en ce que** est utilisé un solvant organique du groupe des alcools en C₁-C₄, de préférence l'éthanol.

13. *N*-(4-benzyloxyphényl)-α-amino-4-benzyloxypropiophénone cristalline de formule 10.

14. Le produit selon la revendications 13, **caractérisé par** les valeurs des angles de diffraction caractéristiques 2θ suivant du spectre de diffraction aux rayons X de poudres (XRPD): 6,71; 19,00, 19,13; 23,63.

15. Utilisation de N-(4-benzyloxyphényl)-α-amino-4-benzyloxypropiophénone de formule 10 pour la préparation de 5-benzyloxy-2-(4-benzyloxyphényl)-3-méthyl-1H-indole de formule 1.

16. Utilisation de N-(4-benzyloxyphényl)-α-amino-4-benzyloxypropiophénone de formule 10 pour la préparation de 2-(4-hydroyphényl)-1-[4-(2-azépan-1-yl-éthoxy)-benzyl]-3-méthyl-1H-indole-5-ol (bazedoxifene) de formule 2.
